(19) Europäisches Patentamt
European Patent Office
Office européen des brevets

(11) Publication number: **0 133 767 B1**

## EUROPEAN PATENT SPECIFICATION

(45) Date of publication of patent specification: 03.04.91   (51) Int. Cl.⁵: **A61K 37/66**

(21) Application number: **84304992.5**

(22) Date of filing: **23.07.84**

(54) **Gamma interferon composition.**

(30) Priority: 04.08.83 JP 143484/83
29.08.83 JP 157560/83

(43) Date of publication of application:
**06.03.85 Bulletin 85/10**

(45) Publication of the grant of the patent:
**03.04.91 Bulletin 91/14**

(84) Designated Contracting States:
**BE DE FR GB NL SE**

(56) References cited:
EP-A- 0 077 670
EP-A- 0 080 879
EP-A- 0 087 686
EP-A- 0 110 044

(73) Proprietor: **GREEN CROSS CORPORATION**
**15-1, Imabashi-1-chome Higashi-ku Osaka-shi**
**Osaka 541(JP)**

(72) Inventor: **Noda, Munehiro**
**Tacho**
**Tenri-Shi(JP)**
Inventor: **Fujita, Takaaki**
**1-17, Minamishimizucho-3-Cho**
**Sakai-Shi(JP)**
Inventor: **Morise, Hiroshi**
**5-4-1307, Kuzuhahanazonocho**
**Hirakata-Shi(JP)**
Inventor: **Arimura, Hirofumi**
**18-1-401, Uenosaka-2-Chome**
**Toyonaka-Shi(JP)**
Inventor: **Suyama, Tadakazu**
**3-7, Matsuigaoka-4-Chome**
**Tanabecho Tsuzuki-Gun Kyoto(JP)**

(74) Representative: **Vossius & Partner**
**Siebertstrasse 4 P.O. Box 86 07 67**
**W-8000 München 86(DE)**

## Description

This invention relates to a stable gamma interferon (hereinafter referred to as IFN-$\gamma$) composition.

IFN-$\gamma$ is a glycoprotein having a molecular weight of about 20,000 to 40,000 which is produced when human leukocytes are stimulated with a mitogen such as phytohemagglutinin (hereinafter referred to as PHA) or concanavalin A (hereinafter referred to as Con-A) used as an inducer.

IFN-$\gamma$ has a marked immunosuppressive, anti-viral and anticellular action; further, it causes a synergistic effect with IFN-$\alpha$ and IFN-$\beta$; moreover, it has 10 to 100 times as high an anti-proliferation effect on tumor cells as that of IFN-$\alpha$ or IFN-$\beta$. Accordingly, the usefulness of IFN-$\gamma$ as a medicine seems to be very promising.

IFN-$\gamma$ is unstable under acid conditions (for example, pH of about 2) or to heat. It also has poor storage stability in solution, and during lyophilization.

From EP-A-80 879 it is known to stabilize IFN-$\alpha$ from leukocytes or the corresponding recombinant IFN-$\alpha$ with a trihydric or higher polyhydric sugar alcohol such as glycerol or mannitol, optionally together with e.g. an acidic sugar or its salt such as ascorbic acid; see claims 1 to 4.

IFN-$\alpha$ consists of multiple species. In SDS-polyacrylamide gel electrophoresis the molecular weight ranges from 15,000 to 21,000. IFN-$\alpha$ is largely devoid of carbohydrate, i.e. it is non-glycosylated. IFN-$\alpha$ contains about 166 amino acid units. There is no significant homology (i.e. degree of identity) between IFN-$\alpha$ and IFN-$\gamma$ on the DNA-level.

Concretely, EP-A-80 879 discloses a mixture of 0.1 $\mu$l of a $10^5$ IU/ml interferon solution and 0.9 ml of a 45 weight % sugar alcohol solution; see Experiment 1. This corresponds to about 400 mg sugar alcohol to $10^4$ IU interferon. Thus a huge amount of sugar alcohol is added to interferon.

The primary sequence of IFN-$\gamma$ (immune interferon) consists of 146 amino acids. It is glycosylated; glycosylation sites are at position 28 and 100. This protein cannot be compared with IFN-$\alpha$; see below, item 2.1.

From EP-A-77 670, page 41, it is known to prepare liquid pharmaceutical compositions for parenteral administration containing IFN-$\gamma$ and human serum albumin. The solution containing 3 mg IFN-$\gamma$ of "specific activity of, e.g. 2 x $10^8$ U/mg" in 25 ml 5 N human serum albumin solution is preferably stored in the cold (-20$^\circ$ C) prior to use.

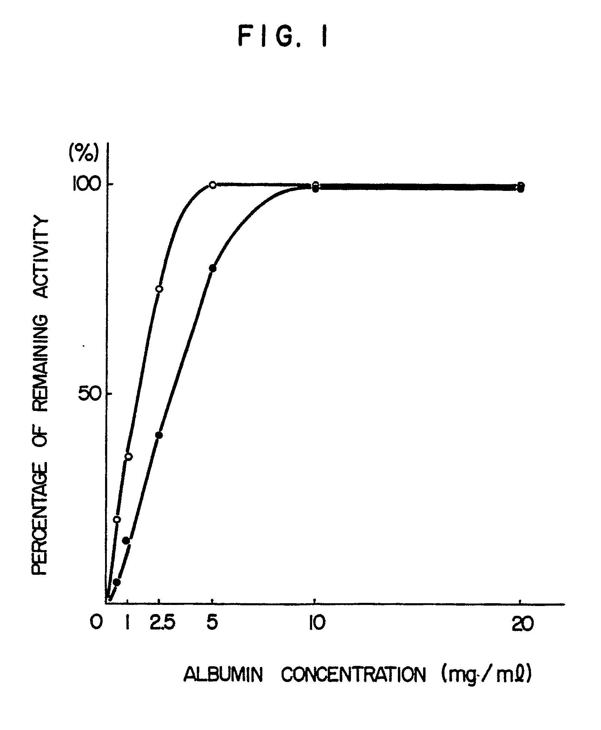

Concretely, EP-A-77 670 discloses a mixture of 3 mg IFN-$\gamma$ having a relative activity of 2 x $10^8$ IU/mg and 25 ml 5 N albumin solution. I "5 N" is considered to be "5 (w/v%)", the mixture should contain 6 x $10^8$ IU of IFN-$\gamma$ and 1,250 mg albumin, which corresponds to 0.021 mg albumin per $10^4$ IU IFN-$\gamma$. Thus the concentration of albumin is much lower than according to claim 1 of the present invention. As is apparent from Experimental Example 2 and Figure 1 of the present application, such a small amount of albumin as disclosed in EP-A-77 670 does not give a stabilizing effect.

EP-A-87 686 is a reference to be considered only in connection with Art. 54(3) EPC.

It describes a lyophilized preparation; see page 14. Each vial contains approximately 15 mcg of homogeneous IFN-$\gamma$ having presumably an activity of 1 x $10^8$ units/mg protein (see page 8, lines 22 to 25) and 5 mg human serum albumin. Thus it discloses a mixture of 15 x $10^5$ IU IFN-$\gamma$ and 5 mg albumin. This corresponds to 0.03 mg albumin per $10^4$ IU IFN-$\gamma$. The albumin concentration is much lower than according to claim 1 of the present invention.

EP-A-110 044 is to be considered only in connection with Art. 54(3) EPC. It describes pharmaceutical compositions containing the human IFN-$\gamma$ protein and other physiologically acceptable inert components such as a salt, diluent, adjuvant, another carrier, buffer, binding agent, surfactant and preservatives; see page 13, lines 7 to 17. In Example 7 on page 24, an injectable preparation is prepared from 5 mg human IFN-$\gamma$ (specific activity 2 x $10^7$ U/mg) in 100 ml of 5% HSA. The solution is lyophilized. This means a mixture of 1 x $10^8$ IU IFN-$\gamma$ and 5 g albumin which corresponds to 0.5 mg albumin per $10^4$ IU IFN-$\gamma$. Thus the albumin content is much lower than according to claim 1 of the present invention.

Thus, the technical problem underlying the present invention is to provide a stable IFN-$\gamma$ composition. The solution to this problem is based on the surprising finding that the addition of a definite amount of a sugar or an albumin to IFN-$\gamma$, causes stabilisation of IFN-$\gamma$. No inactivation takes place during the lyophilization of an aqueous solution containing IFN-$\gamma$. Moreover the storage stability of the dry preparation formed by lyophilization is improved.

Thus, this invention relates to a stable IFN-$\gamma$ composition which comprises IFN-$\gamma$ and a sugar in an amount of 5-10 mg or an albumin in an amount of 10-20 mg per 1x$10^4$ units IFN-$\gamma$.

The IFN-$\gamma$ to be used in this invention is usually obtained by purification by chromatography using an antigen column or the like of the crude IFN-$\gamma$ produced by stimulation of human leukocytes with a mitogen such as PHA and Con-A used as an inducer. But a wide variety of other IFN-$\gamma$ including those produced by E.coli , yeast and the like by virtue of genetic engineering may also be used irrespective of their origin.

The sugars to be used in this invention include not only normal sugars such as monosaccharides and disaccharides, but also sugar alcohols and mixtures thereof. Examples of monosaccharides are glucose, mannose, galactose and fructose. Examples of disaccharides are sucrose, maltose and lactose. Examples of sugar alcohols are mannitol and xylitol. A sugar may be used in admixtures with an albumin.

The albumin to be used in this invention is preferably of human origin because of the antigenicity problem. Such albumins can be used without further limitation as long as they have been purified for medical use. The purity is preferably such that they contain at least 80% of albumin as determined by electrophoresis analysis. As methods for obtaining human albumin, there may be mentioned, for example, the ethanol fractionation method (Japanese Patent Publication Nos. 2869/72 and 5297/60) and the method of heating a fraction thereof in the presence of an organic acid (Japanese Patent Publication Nos. 1604/68 and 401321/76). Albumins which have been subjected to heat treatment (preferably at 60°C for about 10 hours) to effect inactivation of hepatitis virus and the like are preferably used.

The amount of sugar or albumin sufficient for stabilizing IFN-$\gamma$ is in a ratio of at least 5 mg of sugar or at least 3 mg of albumin per $1 \times 10^2 - 1 \times 10^7$ units of IFN-$\gamma$ , and preferably 10 mg of sugar or 5-10 mg of albumin per $1 \times 10^4 - 1 \times 10^5$ units of IFN-$\gamma$. This compounding ratio is applicable to the stabilization of IFN-$\gamma$ in the form of either solid or aqueous solution. In the lyophilization of an aqueous solution of IFN-$\gamma$, addition of sugar to a concentration of at least 5 mg/ml, preferably 10 mg/ml of sugar or at least 3 mg/ml, preferably 5 mg/ml of albumin in the solution is sufficient to achieve the stabilization of IFN-$\gamma$, irrespective of the content of IFN-$\gamma$ in the solution.

The lyophilization in the presence of a sugar or an albumin may be performed, for example, as follows. An aqueous solution containing purified IFN-$\gamma$ is adjusted to pH 5 to 9. The sugar or albumin is added to the solution in a stabilizing amount mentioned above; the resulting aqueous solution is filtered aseptically, dispensed in portions into vials, and then lyophilized in a conventional manner.

The lyophilized preparation of IFN-$\gamma$ thus obtained has preferably a composition approximately corresponding to 10,000 - 500,000 units (preferably 50,000 - 500,000 units) of IFN-$\gamma$, 5-20 mg (preferably 10-20 mg) of sugar or 3-20 mg (preferably 5-20 mg) of albumin and 9 - 18 mg of sodium chloride.

The IFN-$\gamma$ preparation provided by the method of this invention is administered orally or parenterally. The dose varies depending upon the diseases to be treated, administration routes and so forth. When used as injections, for example, it is usually made up into a solution containing 1-10% (w/v) of IFN-$\gamma$ with distilled water or physiological saline solution for injection, and administered intravenously or intra-muscularly at a dose of $2 \times 10^3 - 1 \times 10^4$ units of IFN-$\gamma$ /kg body weight depending on the symptoms of the patients. In which case albumin would be administered in an amount of 0.1-0.4 mg/kg and sugar in an amount of 0.2-0.4 mg/kg.

This invention will be described in more detail below with reference to Examples and Experimental Examples.

The unexpected and surprising effect of the IFN-$\gamma$ composition of the present invention is evident from Experimental Example 1 and Table 1 and Experimental Example 2 and Figure 1. This effect cannot be expected from the teaching disclosed in EP-A-80 879 and 77 670.

Examples 1 to 9

IFN-$\gamma$ produced in leukocytes with PHA used as the stimulant was purified by subjecting it to an affinity chromatography using Sepharose-monoclonal IFN-$\gamma$ antibody as column and 2M KSCN solution (pH 8) as eluent to give a specific activity of $10^6$ U/mg or more. The purified IFN-$\gamma$ was dialyzed against a phosphate-sodium chloride buffer solution, pH 7. Then, a series of solution groups of various titers was prepared which con tained the dialyzed IFN-$\gamma$ in concentrations of $1 \times 10^2$ units/ml, $1 \times 10^3$ units/ml, $1 \times 10^4$ units/ml, $1 \times 10^5$ units/ml and $1 \times 10^6$ units/ml. To each of the solution groups, glucose (Example 1), mannose (Example 2), galactose (Example 3), fructose (Example 4), sucrose (Example 5), maltose (Example 6), lactose (Example 7), mannitol (Example 8) or xylitol (Example 9) was added to a concentration of 10 mg/ml, respectively. The solutions were then filtered aseptically, dispensed in 2 ml portions into vials of 10 ml volume, and lyophilized at temperatures finally reaching 25°C.

The water content of the resulting lyophilized product determined according to "the general method, criterion for biological preparations" was found to be about 0.2% for every specimen. All of the lyophilized products dissolved immediately on addition of 2 ml of distilled water for injection and gave a clear, colorless solution. The amounts of remaining IFN-$\gamma$ determined with these solutions did not differ from those before lyophilization.

Example 10.

Example 1 was repeated, provided that albumin was added at a concentration of 5 mg/ml in place of glucose at a concentration of 10 mg/ml.

The resulting lyophilized product has a water content of about 2% and dissolved immediately. The property of the solution and the activity of remaining IFN-$\gamma$ therein are the same as in Example 1.

Experimental Example 1

A series of experiments was carried out to confirm the stabilizing effect according to this invention. To a solution of purified IFN-$\gamma$ ($1 \times 10^4$ units/ml; the titer being determined according to the CPE inhibition method in FL-Sindvis Virus system),glucose, sucrose, or mannitol was added to concentrations of 1 mg/ml, 5 mg/ml and 10 mg/ml, respectively, and the resulting solutions were lyophilized. The titer of the product was determined immediately before lyophilization, immediately after lyophilization, and after storage of 6 months at room temperature. The percentage of remaining activity relative to the titer immediately after the addition of sugar was as shown in Table 1.

## Table 1

| Sugar | | Remaining ratio of IFN-$\gamma$ activity (%) | | |
|---|---|---|---|---|
| Kind | Added amount | Immediately before lyophili-zation | Immediately after lyophili-zation | 6 Months' storage at room tem-perature |
| Glucose | 1 | 98 | 40 | 19 |
| | 5 | 102 | 82 | 76 |
| | 10 | 101 | 93 | 89 |
| Sucrose | 1 | 99 | 46 | 15 |
| | 5 | 97 | 86 | 78 |
| | 10 | 100 | 96 | 90 |
| Mannitol | 1 | 100 | 37 | 14 |
| | 5 | 101 | 83 | 75 |
| | 10 | 99 | 91 | 87 |
| None | | 100 | — | 32 |

4

EP 0 133 767 B1

Experimental Example 2

A series of experiments was carried out to confirm the stabilizing effect according to this invention. To a solution of purified IFN-$\gamma$ (1 x $10^4$ units/ml; the titer being determined according to the CPE inhibition method in FL-Sindvis Virus system), human serum albumin to various concentrations (0 to 20 mg/ml as shown in Fig. 1) was added, and the resulting solutions were lyophilized. The titer of the lyophilized product was determined immediately after lyophilization (indicated by symbol O ) and after storage of 6 months at room temperature (indicated by symbol ● ). The percentage of the remaining activity relative to the titer immediately after the addition of human serum albumin was as shown in Fig. 1.

**Claims**

1. A stable gamma interferon composition comprising a gamma interferon and a sugar in an amount of 5 - 10 mg or an albumin in an amount of 10 - 20 mg per 1 x $10^4$ units (IU)gamma interferon.

2. The gamma interferon composition of Claim 1, wherein the sugar is a monosaccharide, a disaccharide or a sugar alcohol.

3. The gamma interferon composition of Claim 2, wherein the sugar is glucose, mannose, galactose, fructose, sucrose, maltose, lactose, mannitol or xylitol.

4. The gamma interferon composition according to any one of Claims 1 to 3 in the form of a lyophilized solid.

5. The stable gamma interferon composition according to any one of Claims 1 to 3 in the form of an aqueous solution.

6. A process for producing the gamma interferon composition of Claim 4, which comprises lyophilizing the gamma interferon composition of Claim 5.

**Revendications**

1. Composition stable à base d'interféron gamma, comprenant un interféron gamma et un sucre selon une quantité de 5 à 10 mg, ou une albumine selon une quantité de 10 à 20 mg pour 1 x $10^4$ unités (UI) d'interféron gamma.

2. Composition à base d'interféron gamma selon la revendication 1, dans laquelle le sucre est un monosaccharide, un disaccharide ou un alcool glucidique.

3. Composition à base d'interféron gamma selon la revendication 2, dans laquelle le sucre est le glucose, le mannose, le galactose, le fructose, le saccharose, le maltose, le lactose, le mannitol ou le xylitol.

4. Composition à base d'interféron gamma selon l'une quelconque des revendications 1 à 3, sous la forme d'un solide lyophilisé.

5. Composition stable à base d'interféron gamma selon l'une quelconque des revendications 1 à 3, sous la forme d'une solution aqueuse.

6. Procédé de préparation de la composition à base d'interféron gamma de la revendication 4, selon lequel on lyophilise la composition à base d'interéfon gamma de la revendication 5.

**Ansprüche**

1. Stabile gamma Interferon-Zusammensetzung, die ein gamma Interferon und einen Zucker in einer

5

Menge von 5 - 10 mg oder ein Albumin in einer Menge von 10 - 20 mg pro 1 x 10$^4$ Einheiten (IU) gamma Interferon umfaßt.

2. Gamma Interferon-Zusammensetzung nach Anspruch 1, wobei der Zucker ein Monosaccharid, ein Disaccharid oder ein Zuckeralkohol ist.

3. Gamma Interferon-Zusammensetzung nach Anspruch 2, wobei der Zucker Glucose, Mannose, Galactose, Fructose, Saccharose, Maltose, Lactose, Mannit oder Xylit ist.

4. Gamma Interferon-Zusammensetzung nach einem der Ansprüche 1 bis 3 in Form eines lyophylisierten Feststoffes.

5. Stabile gamma Interferon-Zusammensetzung nach einem der Ansprüche 1 bis 3 in Form einer wäßrigen Lösung.

6. Verfahren zur Herstellung der gamma Interferon-Zusammensetzung nach Anspruch 4, das die Lyophilisation der gamma Interferon-Zusammensetzung nach Anspruch 5 umfaßt.

FIG. I